Europäisches Patentamt

European Patent Office

Office européen des brevets

(19)

(11) Veröffentlichungsnummer: **0 358 767**

**A1**

(12)
# EUROPÄISCHE PATENTANMELDUNG
## veröffentlicht nach Art. 158 Abs. 3 EPÜ

(21) Anmeldenummer: 89900364.4

(22) Anmeldetag: 27.01.88

(86) Internationale Anmeldenummer: PCT/SU88/00021

(87) Internationale Veröffentlichungsnummer: WO 89/06984 (10.08.89 89/18)

(51) Int. Cl.⁵: **A61M 25/00**

(43) Veröffentlichungstag der Anmeldung: 21.03.90 Patentblatt 90/12

(84) Benannte Vertragsstaaten: CH DE FR GB IT LI SE

(71) Anmelder: **KIEVSKY NAUCHNO-ISSLEDOVATELSKY INSTITUT NEIROKHIRURGII**
ul. Manuilskogo, 32
Kiev, 252655(SU)

(72) Erfinder: **SCHEGLOV, Viktor Ivanovich**
ul. Belorusskaya, 15-51
Kiev, 252119(SU)
Erfinder: **SAVENKO, Alexandr Grigorievich**
ul. Murashko, 5-100
Kiev, 252050(SU)
Erfinder: **ROMODANOV, Sergei Andreevich**
ul. Artema, 8-56
Kiev, 252036(SU)
Erfinder: **SERBINENKO, Fedor Andreevich**
Kutuzovsky pr., 33-43
Moscow, 121165(SU)
Erfinder: **KOBTSEV, Alexei Vasilievich**
ul. Krasnoarmeiskaya, 9-41 Khmelnitskaya obl.
Kamenets-Podolsky, 281900(SU)

(74) Vertreter: **Lehn, Werner, Dipl.-Ing. et al**
**Hoffmann, Eitle & Partner Patentanwälte**
Arabellastrasse 4
D-8000 München 81(DE)

(54) **OKKLUSIONSANORDNUNG.**

(57) Okkludierende Vorrichtung, die einen aufblasbaren, vermittels Sphinkters (3) mit dem einsetzbaren Katheter (4) verbundenen Ballon (1) vorsieht, wobei die Wandung des erwähnten Ballons (1) mindestens eine ringförmige Verdickung an dem Sphinkter (3) herum trägt.

FIG.1

EP 0 358 767 A1

OKKLUDIERENDE VORRICHTUNG

Technisches Gebiet

Die vorliegende Erfindung bezieht sich auf das Gebiet Medizin, insbesondere auf Röntgenchirurgie, und zwar auf okkludierende Vorrichtungen und kann bei der Behandlung verschiedenartiger Gefäßerkrankungen, insbesondere der Gefäßaneurysmen und bei der Durchführung der rekonstruktiven Gefäßoperationen verwendet werden.

Zugrundeliegender Stand der Technik

Gegenwärtig stellen die Erkrankungen der Gehirngefäße, des Herzens und anderer Organe die verbreitesten Erkrankungen des Menschen dar. Zu den wirksamsten Behandlungsverfahren der Gefäßerkrankungen zählen die chirurgischen Verfahren der Heilbehandlungen. Jedoch bei der ausreichenden Wirksamkeit der chirurgischen Behandlungsverfahren zeichnet sich die Durchführung derselben durch ihre hohe Verletzbarkeit, verwickelte Schwierigkeit und durch hohe Kosten aus. Die Durchführung der chirurgischen Operationen an Gehirngefäßen wird des öfteren mit den Komplikationen begleitet, die mit den zusätzlichen Verletzungen der lebenswichtigen Strukturen des Gehirnes zusammenhängen.

Zur letzten Zeit gewinnt die Röntgen- oder endovaskuläre Chirurgie immermehr an ihrer Verbreitung, die sich bei einer geringen Verletzbarkeit, guten Beherrschbarkeit und bei den möglichkleinsten Nachoperationskomplikationen auswirkt.

Die Schaffung und Weiterentwicklung der Röntgenchirurgie aber bedarf der Erbauung von grundsätzlich neuen, einzigartigen Vorrichtungen zur Durchführung der Operationen.

Bekannt ist ganze Reihe der Vorrichtungen zur Durchführung der röntgenchirurgischen Operationen an Gefäßen wie beispielsweise Katheter mit abnehmbaren Ballonen kleineren Abmessungen (FR, A, 2 361 124). Eine bekannte Vorrichtung besteht aus einem über das Übergangsstück mit dem abnehmbaren Ballon verbundenen Katheter. Die betreffenden abnehmbaren Ballone weisen unterschiedliche Ausführungsformen, und zwar mit einer Verdickung der gegenüber dem Sphinkter des Ballons liegenden Wandung, mit einer selbstverschließenden Öffnung in der Verdickungswandung gegenüber den Ballonsphinkter auf. Der Gebrauch des Ballonkatheters der betreffenden Ausführung besteht in dessen Einführung in das Gefäß durch die Punktionsnadel und in dessen Hinanschiebung an die pathologische Stelle des jeweiligen Gefäßes mit nachfolgender vollkommener Okklusion des pathologischen Gefäßes durch Aufblasen des Gefäßes und dessen Auffüllung mit einem schnellerstarrenden Stoff sowie mit der Entfernung des Katheters. Die betreffende Vorrichtung ist infolge deren mangelhafter, aufbaumäßiger Ausführung in ihrer Anwendung sehr eingeschränkt und ermöglicht nur eine vollkommene Ausschaltung eines Gefäßes aus dem Blutkreislauf. Die mit Hilfe der betreffenden Vorrichtung durchzuführenden Operationen werden mit den Nachoperationskomplikationen begleitet, die die physiologischen Störungen im Kreislauf bewirken.

Eine andere, zu dem Erfindungsgedanken nahliegende, okkludierende Vorrichtung stellt ebenfalls einen Katheter mit dem abnehmbaren Ballon dar (GB, A, 2 115 239). Diese Vorrichtung besteht aus einem aufblasbaren Ballon, der vermittels eines Übergangsstückes mit dem Katheter verbunden ist. Auf dem Übergangsstück ist eine Spirale aufgewickelt, die über einen Verbindungsdraht oder zwei -drähte mit einer Stromquelle verbunden ist. Die Abtrennung des Ballons von dem Katheter erfolgt vermittels der Durchbrennung oder Verschmelzung des verbindenden Übergangsstücks.

Die Behandlung der Erkrankungen von Gefäßen mit Hilfe der erwähnten Vorrichtung wird wie folgt durchgeführt. Nach der Durchführung der Vorrichtung durch die Punktionsnadel wird diese in dem Gefäß an die pathologische Stelle des Gefäßes hinangeschoben, wonach der Ballon unter Auffüllung mit dem schnellerstarrenden Stoff aufgeblasen wird, indem dadurch eine vollkommene Okklusion des betreffenden Gefäßes zustandekommt. Hiernach wird der Strom aus der Stromquelle zu der Spirale zugeführt, welches das Katheter und den Ballon verbindende Übergangsstück verschmelzt oder durchbrennt, während nach der Abtrennung der Katheter mit der

Spirale und den Verbindungsdrähten zusammen herausgezogen wird.

Der Ballon aber weist hierbei eine starre Konstruktion auf und gibt keine Möglichkeit, in den Aneurismasinnenraum und in die Abzweiggefäße hineinzudringen, wodurch dessen Anwendung eingeschränkt ist. Dieser Ballon kommt bei der vollkommenen Okklusion der großen arteriellen Gefäße zur Anwendung, trotzdem bewirkt die Unvollkommenheit der aufbaumäßigen Ausführung der Befestigung und Abtrennung des Ballons in einigen Fällen die schweren Komplikationen.

Eine zu der erfindungsgemäßen am nächsten stehenden Okklusionsvorrichtung, die an ihrer Anwendung eines weitgehendste Verbreitung gewonnen hat, vertritt Debrun-Ballonkatheter (FR, A, 2 383 673). Dieser Debrun-Ballonkatheter ist aus dem Katheter selbst und einem an diesem aufgesetzten, abnehmbaren, aufblasbaren Ballon mit der in diesem eingebauten Röntgenkontrastmarke, die einen in der gegenüber dem Sphinkter befindlichen Wandungsverdickung des Ballons steckenden Metallklipps darstellt, ausgeführt. Der Sphinkter des Ballons weist seine Krümmung in den Innenraum auf und wird an der Außenwandung des Katheters aufgesetzt. Der erwähnte Katheter als Hauptkatheter trägt noch einen Schiebekatheter darauf, welcher auf den Hauptkatheter oberhalb aufgeschoben wird. Nach der Ausschaltung aus dem Blutkreislauf eines Aneurismasgefäßes wird der Ballon von dem Hauptkatheter abgetrennt, der hiernach mit dem Schiebekatheter zusammen aus dem betreffenden Gefäß herausgenommen wird.

Die Ausschaltung der pathologischen Gefäßstrecke aus dem Blutkreislauf wird wie folgt durchgeführt. Zunächst wird der Debrun-Ballonkatheter durch die Punktionsnadel in das Gefäß eingeführt und an die pathologische Gefäßstrecke herangeschoben. Hiernach wird der Ballon selbst ausgeblasen und mit dem schnellerstarrenden Stoff ausgefüllt. Der Sphinkter an dem Ballon beginnt bei dem Aufblasen sich nach außen umzuspülen, wodurch die Verbindungsstärke des Ballons mit dem Katheter abnimmt. Um den Abtrennungsvorgang zwischen dem Ballon und Hauptkatheter zu vollenden, wird ein anderer Zusatzkatheter auf den den Ballon tragenden Hauptkatheter

aufgesetzt und innerhalb des Gefäßes zu dem Ballon hineingeschoben. Bei diesem Vorschub wird der aufgeblasene Ballon
in der Gefäßhöhle sicher zurückgehalten. Bei der Abnahme des
Ballons wird der Hauptkatheter vermittels des Bewegungsganges
in Richtung dessen Herausnahme aus dem Gefäßkanal unter der
Umstülpung des Sphinkters nach außen herausgezogen. Zugleich
wird der andere Zusatzkatheter in Richtung dem Ballon zu
verschoben, als ob er den Ballon von dem Hauptkatheter weg
abschieben möchte. Nach der Abtrennung des Ballons von dem
Katheter werden beide Katheter aus dem Gefäß herausgenommen.
Der Debrun-Ballonkatheter aber weist eine starre, aufbaumäßige Ausführung auf, die kein Hineindringen des Ballons in die
Abzweiggefäße und in die Aneurysmahöhle hin ermöglicht, wodurch dessen Anwendungsbereich verengt wird, während die
mit Hilfe des Debrun-Ballonkatheters durchgeführten Operationen in der Regel auf eine stationäre Okklusion der zuleitenden Gefäße und der die Aneurysmen tragenden Gefäße eingerichtet werden, wodurch eine weitgehende Veränderung der
Blutversorgung und erschwerte Nachoperationskomplikationen
auftreten.

Darüber hinaus müssen merkwürdige Kraftbelastungen bei
der Abtrennung des Ballons von dem ihn tragenden Katheter
angelegt werden, was auch die Gefahr mit sich bringt. Bei
den gelungenen Versuchen, die Höhle der großen Aneurysmen
bzw. der Aneurysmen mit dem breiten Hals zwecks deren Ausschaltung aus dem Blutstromlauf mit dem Debrun-Ballonkatheter
zu erreichen, müssen bis etwa 10 Ballone in die Aneurysmahöhle eingeführt werden. Bei ihrem Befinden innerhalb der
Aneurysmahöhle verbleiben die Ballons sowohl gegeneinander
als auch gegen die Aneurysmawandung nicht festgelegt, was
deren Wanderung innerhalb der Aneurysmahöhle und Herausschwemmung aus dieser durch den Blutstrom bewirkt, welche
Erscheinungen ihrerseits zu den schwersten Nachoperationskomplikationen wie unterschiedlichstarke Ischämie und Organen- sowie Gewebennekrose, verschiedenartige Sprach- und Bewegungsstörungen der unteren und oberen Extremitäten, Störungen an den verwickelten Arten der Empfindlichkeit in mehreren Fällen mit dem tödlichen Ausgang führen. Die aus dem Blutstrom ausgeschalteten Gefäßaneurysmen rezidivieren dabei mit den wiederholten Blutungen.

Die Möglichkeiten, die obenbeschriebenen Ballonkatheter auszunutzen, werden durch die Starrheit deren aufbaumäßigen Ausführung eingeschränkt, die keine Hineindringung hinter die Krümmungen der Kopfschlagader (Siphone) ermöglicht. Die Gestaltung der Ballons ist derart ausgeführt, daß der Ballon dadurch in die Aneurysmenhöhle schwer einzuführen ist. In diesem Zusammenhang wird die Benutzung der betreffenden Ballonkatheter zum Ausschalten der sackförmigen Aneurysmen aus dem Blutstrom und die Durchführung der rekonstruktiven Operationen äußerst eingeschränkt.

Offenbarung der Erfindung

Die Aufgabe der vorliegenden Erfindung ist es, eine solche okkludierende Vorrichtung zu entwickeln, die durch ihre aufbaumäßige Ausführung des Ballons die Möglichkeit bietet, die Aneurysmen der kleineren und mittleren Gefäße aus dem Kreislauf auszuschalten und die rekonstruktiven Operationen ohne Verletzung der physiologischen Blutversorgung durchzuführen.

Die gestellte Aufgabe wird dadurch gelöst, daß in der okkludierenden Vorrichtung, die einen abnehmbaren, vermittels Sphinkters mit dem einsteckbaren Katheter verbundenen, aufblasbaren Ballon mit der röntgenkontrasten Marke einschließt, erfindungsgemäß der Ballon mit zumindest einer ringförmigen Verdickung an dessen Wandung versehen ist, die an dem Sphinkter herum ausgeführt ist.

Dadurch besteht die Möglichkeit, unter Benutzung der Turbulenz der Blutströmung in den Blutgefäßen in die Gefäße der zweiten und dritten Ordnung in die sackförmigen Aneurysmen dieser Gefäße hineinzudringen und diese aus dem Kreislauf ohne Verletzung der Physiologie des Kreislaufs in den Regionen dieser Gefäße einwandfrei auszuschalten. In diesem Zusammenhang können die rekonstruktiven Operationen durchgeführt werden, sowie die falschen, traumatischen Aneurysmen aus dem Kreislauf ausgeschaltet werden.

Zweckmäßigerweise ist zumindest eine abgeschlossene, mit dem Klebestoff ausgefüllte Zelle innerhalb des Ballons vorzusehen, in deren Außenwandung eine durchgehende Perforation ausgeführt ist. Dadurch entsteht die Möglichkeit, in die Höhle des Aneurysmas den jeweiligen, thrombenbildenden Klebestoff zu befördern, der eine gesicherte Abtrennung der Aneurysmahöhle selbst der geringen Abmessungen mit dem betreffenden Ballon und dessen zuverlässige Zurückhaltung innerhalb der erwähnten Aneurysmahöhle begünstigt, wodurch die durch die Ausschwemmung des Ballons aus der Aneurysmahöhle durch den Blutstrom und durch die Okklusion der lebenswichtigen Gefäße verursachten Komplikationen vermieden werden.

Der Ballon kann noch mit zumindest einer ringförmigen Zusatzverdickung versehen werden. Diese ringförmige Zusatzverdickung ermöglicht eine Steigerung der Sicherheit bei der Durchführung der Operationen zwecks Ausschaltung der Aneurysmen der mittleren und ausgedehnten Größen aus dem Blutkreislauf, die rezidivierenden Erkrankungen durch Steigerung der Festigkeit der Zurückhalterung des Ballons innerhalb der Aneurysmahöhle zu vermeiden, die auf dem Zusammenkleben einer vergrößerten Fläche des Ballons mit dem Thrombus und der Aneurysmawandung zurückzuführen ist. Auf der Oberfläche des Ballons kann zumindest eine Festhalterungszitze vorgesehen werden, das als ein Hohlvorsprung mit den Seitenbohrungen ausgeführt ist. Diese Zitze bewirkt einen zuverlässigen Verschluß der Aneurysmen der kleineren und mittleren Größen mit einem breiten bzw. verengten Hals zustande, läßt die Ausschwemmung des Ballons aus der Aneurysmahöhle durch Vergrößerung der Haftkraft des Thrombus mit dem Ballon vermeiden, die infolge der Durchdringung des Thrombuskörpers ins Innere des erwähnten Vorsprunges erreicht wird.

Der Ballonkatheter kann auch mit Verzweigungen ausgeführt werden.

Diese Verzweigungen ermöglichen die Durchführung der Röntgenoperationen an den schwerzugänglichen Stellen mit einer großen Langstrecke der Gefäßbahn.

## Kurzbeschreibung der Zeichnungen

Nachstehend wird die Erfindung anhand der Beschreibung der konkreten Ausführungsbeispiele und angelegten Zeichnungen näher erläutert, in denen es zeigt:

Fig. 1 Gesamtansicht der erfindungsgemäßen, okkludierenden Vorrichtung im Längsschnitt;

Fig. 2 erfindungsgemäße, okkludierende Vorrichtung in Wirkung;

Fig. 3 erfindungsgemäße, okkludierende Vorrichtung mit der abgeschlossenen Zelle im Längsschnitt;

Fig. 4 erfindungsgemäße, okkludierende Vorrichtung mit der abgeschlossenen Zelle innerhalb der Aneurysmahöhle;

Fig. 5 erfindungsgemäße, okkludierende Vorrichtung mit ringförmigen Zusatzverdickungen im Längsschnitt;

Fig. 6 erfindungsgemäße, okkludierende Vorrichtung aus der Fig. 5 in der Arbeitsstellung;

Fig. 7 erfindungsgemäße, okkludierende Vorrichtung aus der Fig. 5 innerhalb der Aneurysmahöhle;

Fig. 8 erfindungsgemäße, okkludierende Vorrichtung mit einem Vorsprung, Zitze an der Seitenwandungsfläche;

Fig. 9 erfindungsgemäße, okkludierende Vorrichtung mit der auf der Seitenwandungsfläche hervorspringenden Festhalterungszitze innerhalb der Aneurysmahöhle;

Fig. 10 erfindungsgemäße, okkludierende Vorrichtung mit Abzweigungskatheter, teilweise geschnitten;

Fig. 11 erfindungsgemäße, okkludierende Vorrichtung bei der Ausschaltung des Aneurysmas aus dem vertebrobasilaren Gebiet.

## Beste Ausführungsvariante der Erfindung

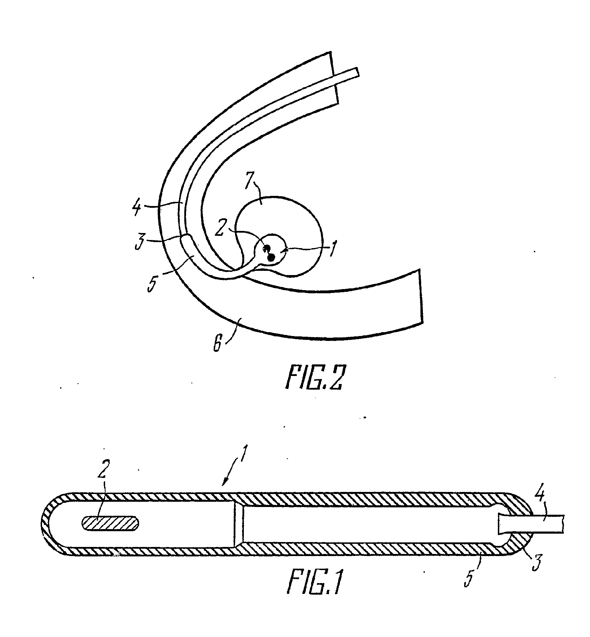

Die erfindungsgemäße, okkludierende Erfindung enthält einen abnehmbaren, aufblasbaren Ballon (Fig. 1) mit der röntgenkontrasten Marke 2, der mit dem einsetzbaren Katheter 4 vermittels Sphinkters 3 verbunden ist. Die Wandung des Ballons 1 ist erfindungsgemäß mit einer an dem Sphinkter 3 herum angebrachten ringförmigen Verdickung 5 versehen. Bei der Einführung des Ballons 1 (Fig. 2) durch das Gefäß 6 in die Aneurysmahöhle 7 wird die Luft in den Innenraum des Ballons 1 durch den Katheter 4 hineingegeben. Dadurch wird das Ende des Ballons 1 aufgeblasen und der Ballon 1 durch den Blutstrom in die Aneurysmahöhle 7 mitgerissen. Das verdickte Ende 5 des Ballons 1 bleibt dabei in seinen Abmessungen unverändert. Hiernach wird das in die Aneurysmahöhle 7 eingeführte Ballonsende mit der Druckluft weiteraufgeblasen und in die Aneurysmahöhle 7 weiter hineingezogen. Die röntgenkontraste Marke 2 verbleibt dabei innerhalb des die Aneurysmahöhle 7 ausfüllenden Ballons 1 frei.

Der Ballon 1 (Fig. 3) der erfindungsgemäßen, okkludierenden Vorrichtung kann zumindest eine abgeschlossene Zelle 8 besitzen. In Fig. 3 ist diese Zelle 8 im Oberteil des Ballons 1 wiedergegeben, trotzdem es muß hier darauf aufmerksam gemacht werden daß diese Zelle 8 an jeder anderen Stelle ausgeführt werden kann, deren Anzahl darüber hinaus über 1 erreichen kann. Die dem Blutstrom zugewandte Außenwandung des Ballons 1 wird mit einer durchgehenden Perforation 9 versehen. Innerhalb der Zelle 8 befindet sich ein Klebstoff 10, durch dessen Eingriff innerhalb der Aneurysmahöhle 7 sich im Blut die Thromben schnell herausbilden und ein zuverlässiges Zusammenkleben des Ballons 1 mit der Wandung des Aneurysmas 7 auftritt.

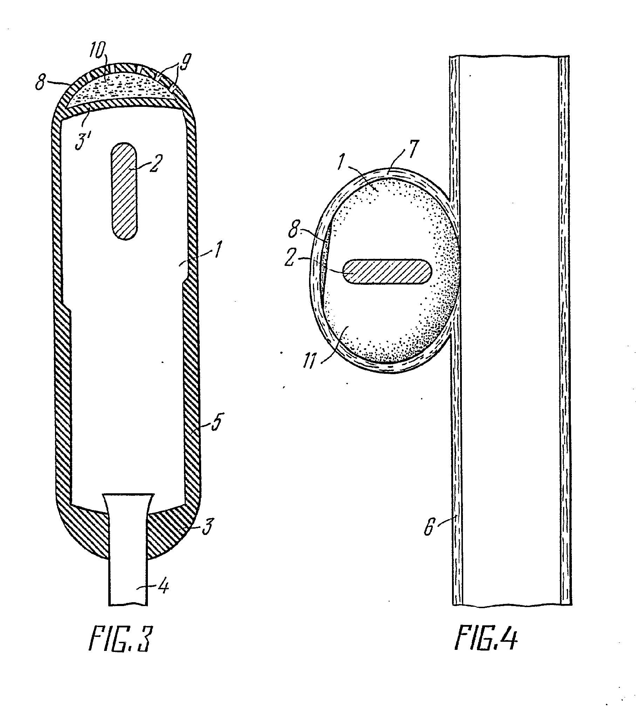

Der in die Aneurysmahöhle 7 eingeführte Ballon 1 (Fig. 4) wird aufgeblasen, in diesen ein schnellerstarrender Stoff 11 hineingeführt. Durch das Aufblasen nimmt der Ballon 1 (Fig. 4) in seinen Abmessungen zu und verformt die abgeschlossene Zelle 8. Bei dieser Verformung der Zelle 8 öffnet sich die durchgehende Perforation 9, durch welche in die Aneurysmahöhle 7 der Klebestoff 10 unter der Zustandebringung eines zuverlässigen Zusammenklebens der Wandung des Aneurysmas 7 und des Ballons 1 heraustritt.

Der Ballon 1 (Fig. 5) der erfindungsgemäßen, okkludierenden Vorrichtung kann noch mit zumindest einer ringförmigen Zusatzverdickung 12 versehen werden. In Fig. 5 und 6

sind zwei erfindungsgemäße, ringförmige, im Oberteil des Ballons 1 vorgesehene Verdickungen dargestellt. Es muß aber vermerkt werden, daß an dem Ballon 1 mehrere ringförmige Zusatzverdickungen 12 in verschiedenen Abständen eine von der anderen angebracht werden können.

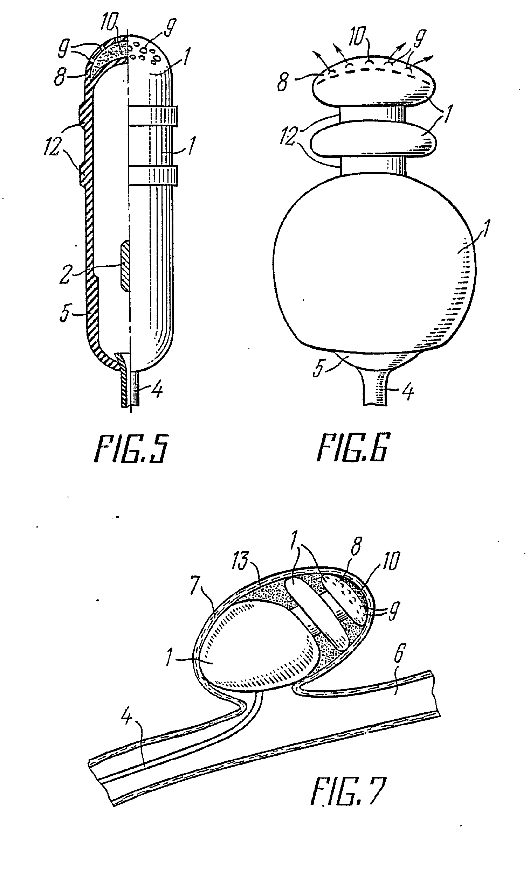

Der in die Aneurysmahöhle 7 (Fig. 7) eingeführte Ballon 1 wird mit dem schnellerstarrenden Stoff 11 ausgefüllt.

Der Ballon 1 erhält unter der dabei stattfindenden Vergrößerung eine bestimmte, durch die Größen der ringförmigen Verdickungen 12 und Zwischenabschnitten dazwischen vorgegebene Form. Die dabei auseinandergezogene Zelle 8 verformt sich unter Öffnung der durchgehenden Perforation 9, durch welche in die Aneurysmahöhle 7 der Klebestoff 10 unter Thrombenbildung in dem innerhalb der Aneurysmahöhle 7 befindlichen Blut 13 heraustritt. Der ausgebildete Blutthrombus klebt dabei an dem Ballon 1 und der Wandung des Aneurysmas 7 unter dessen einwandfreier Ausschaltung aus dem Blutkreislauf.

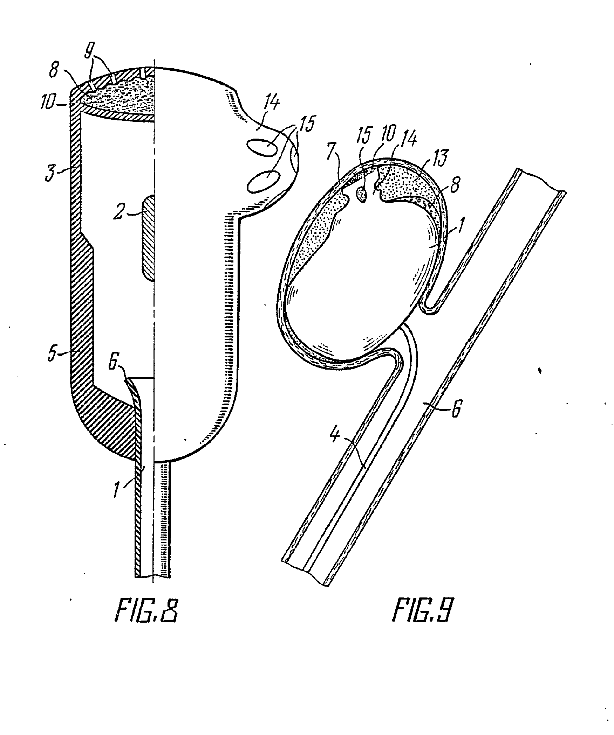

Die erfindungsgemäße, okkludierende Vorrichtung kann weiter mit zumindest einer als ein Hohlvorsprung mit den Seitenöffnungen 15 ausgebildeten Festhalterungszitze 14 ausgestattet werden. In Fig. 8 sind diese hohle Festhalterungszitze 14 und die abgeschlossene Zelle 8 dargestellt, die an der Seitenfläche des Ballons 1 ausgeführt sind. Die Ausführungsform der Festhalterungszitze 14 kann jeweils eine beliebige Gestalt aufweisen, muß aber mit den durchgehenden Löchern 15 versehen hohl sein. Die Anzahl der Festhalterungszitzen 14 und ihre gegenseitige Anordnung sowie Anordnung auf dem Ballon 1 kann verschiedenartig sein.

Nach der Einführung des Ballons 1 in die Aneurysmahöhle 7 (Fig. 9) wird dieser mit dem schnellerstarrenden Stoff 11 ausgefüllt, wobei die Röntgenmarke 2 ihre willkürliche Lage in dem Ballon einnimmt. Bei dessen Auseinanderblasen schließt der Ballon 1 den Hals des Aneurysmas 7 zu, zieht die abgeschlossene Zelle 8 unter der Öffnung der Perforation 9 auseinander und läßt den Klebestoff 10 in die Aneurysmahöhle 7 unter Ansteckung der Herausbildung eines Thrombus 13 herausfließen.

Der entstandene Thrombus 13 klebt an dem Ballon 1, sticht die Festhalterungszitze 14 durch die durchgehenden Seitenlöcher 15 hindurch, indem dadurch die zusätzlichen Verbindungen entstehen, die zuverlässig den Ballon 1 innerhalb der Aneurysmahöhle 7 zurückhalten.

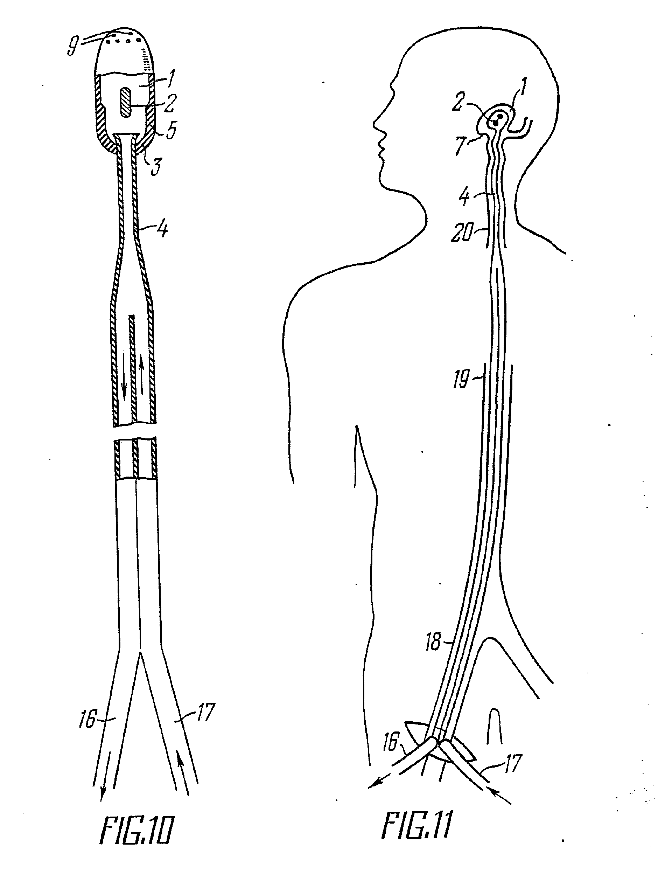

Die erfindungsgemäße, okkludierende Vorrichtung kann auch einen Katheter mit Abzweigungen einschließen. In Fig. 10 ist der Katheder 1 mit zwei Abzweigkanälen 16 und 17 wiedergegeben. Diese Abzweigkanäle 16 und 17 des Abzweigkatheters 4 gehen allmählich in den Einkanalkatheter über. Das Größenverhältnis des Einkanal- und des Abzweigkatheters kann unterschiedliche Werte erreichen.

In die Aneurysmahöhle 7 innerhalb des vertebro-basilaren Gebietes wird der Ballon 1 (Fig. 11) durch die Oberschenkel und weiter durch die Bauchaorta 18 und 19 sowie durch die Wirbelschlagader 20 hineingeführt. Zur Kontrolle der Ausfüllung des Aneurysmas 7 wird ein Röntgenkontraststoff innerhalb des Kanals 16 des Katheters 4 eingeführt, wonach bei dem Erreichen des Ballons 1 mit dem Kontraststoff der Kanal 17 des Katheters 4 zugemacht wird. Zum Herauszug des Röntgenkontraststoffes aus dem Ballon 1 werden beide Abzweigkanäle 16 und 17 des Katheters 4 aufgemacht.

Die Ausfüllung des Ballons mit dem schnellerstarrenden Stoff 11 wird vermittels des Abzweigkanals 17 des Katheters 4 bei dem geöffneten Abzweigkanal 16 des -katheters 4 selbst auch bei einem geringeren Unterdruck in diesem durchgeführt. Nachdem der Ballon 1 mit dem schnellerstarrenden Stoff 11 teilweise ausgefüllt wird, wird der Abzweigkanal 16 des Katheters 4 zugeschlossen, während der Ballon 1 mit dem schnellerstarrenden Stoff weiter ausgefüllt wird, bis er voll ist.

Nach der erfolgten Diagnostik des Aneursysmas der großen Abmessungen mit der gewöhnlichen Größe der Aneurysmasmündung wird der Ballon 1 mit zwei ringförmigen Verdickungen (Fig. 5 und 6) gewählt. Mit einer speziellen Nadel wird Punktion der Arterie durchgeführt. Durch diese Nadel wird die okkludierende Vorrichtung in die Arterie 6 hineingeführt, deren abgeschlossene Zelle 8 mit dem thrombenbildenden Klebestoff 10 vollgefüllt ist, Vorschub an die Mündung des Aneurysmas 7 mit Hilfe der Handhabungen und des Blutstromes verläuft.

Unter Benutzung der Besonderheiten der Hämodynamik des Blutstromes wird nun der Ballon 1 in die Aneurysmahöhle 7 hineingeschoben. Über den Abzweigkatheter 4 wird jetzt der Ballon 1 mit dem Röntgenkontraststoff zum Teil ausgefüllt, während je nach der Ausfüllung mit dem Kontraststoff sowie vermittels der Röntgenkontrastmarke 2 die Kontrolle der Stellungslage des Ballons 1 innerhalb der Aneurysmahöhle 7 durchgeführt wird. Hiernach wird der Röntgenkontraststoff aus dem Ballon 1 über den Abzweigkatheter 4 abgezogen. Nach dem erfolgten Abzug wird in den Ballon 1 der schnellerstarrende Stoff 11 über den Abzweigkatheter 4 vermittels der Spritze eingeführt. Der Ballon 1 nimmt dadurch in seinen Abmessungen zu und füllt die Aneurysmahöhle 7 völlig aus. Die Wandung des Ballons 1 ziehen sich durch den Eingriff der ringförmigen Verdickungen 12 unterschiedlich stark auseinander, wodurch der Ballon 1 eine komplizierte Gestalt (Fig. 6) erhält. Dabei verformt sich die abgeschlossene Zelle 8, die im voraus mit dem thrombenbildenden Klebestoff 10 vollgefüllt ist. Dieser thrombenbildende Klebestoff 10 tritt in die Aneurysmahöhle 7 durch die Perforation 9 und bildet dort einen den Körper des Ballons 1 unter dessen zuverlässigem Zurückhalten innerhalb der Aneurysmahöhle 7 umfassen Thrombus 13 (Fig. 7). Der Abzweigkatheter 4 hält den Ballon 7 vermittels des Sphinkters 3 zurück. Durch leichte Handbewegungen, leichte Züge am Katheter 4 wird dieser aus dem Ballon 1 herausgezogen, während der Sphinkter 3 zuschließt.

Zum Unterschied von allen bekannten Ballonkathetern ermöglichen die erfindungsgemäßen, okkludierenden Vorrichtungen aus der Blutströmung die artiellen Aneurysmen jeweils beliebiger Lokalisation auszuschalten, wenn deren Abmessungen im Durchmesser 0,5 mm übertreffen und der Aneurysmahals zur Einführung des Ballons in die Aneurysmahöhle zugänglich ist.

## Gewerbliche Verwertbarkeit

Es ist von dem außerordentlichen Wert, daß mit Hilfe der

erfindungsgemäßen, okkludierenden Vorrichtungen in meisten Fällen eine rekonstruktive, d. h. eine ideale Operation, und zwar die Okklusion der Höhle eines arteriellen Aneurysmas, deren Ausschaltung aus der Blutströmung durchgeführt werden und das Durchflußvermögen der das Aneurysma tragenden Arterie des Gehirns und anderer Organe aufrechterhalten kann. Die großen und mehrjährig angesammelten Erfahrungen der Behandlung von Kranken mit den Aneurysmen haben bestätigt, daß deren endovaskuläre Ausschaltung eine physiologischgerechteste, hochwirksame und zuverlässige darstellt und mit der möglichstkleinen Operationsverletzung durchgeführt wird, und zwar die Erhaltung des elterlichen Gefäßes und die Okklusion der Aneurysmahöhle bringen einen qualitätsneuen Sinn der endovskulären Operationen bei den arteriellen Aneurysmen mit sich.

Mit Hilfe der erfindungsgemäßen, okkludierenden Vorrichtungen gelingt es, die rekonstruktiven Operationen selbst bei den verschiedenartigen, karotidenkavernösen Anastomosen durchzuführen, die verschiedenartigen arteriellen Sinusanastomosen erfolgreich auszuschalten, die Behandlung der Gefäßaneurysmen der Organe in die Bauchhöhle sowie der Aorta durchzuführen.

Die mit Hilfe der erfindungsgemäßen, okkludierenden Vorrichtungen mit den abzutrennenden Ballonkatheter an den traumatisch-falschen Aneurysmen des kavernösen Gebiets der inneren Kopfschlagader durchzuführenden, endovaskulären Operationen geben die Möglichkeit, bei der Mehrzahl von Kranken die rekonstruktive Operation durchzuführen und sind von der Auswahloperation geworden, da bei diesen Aneurysmen die rekonstruktive Operation nur unter Benutzung der abzutrennenden Ballonkatheter durchgeführt werden kann.

Bei Verwendung der leichtabzutrennenden Ballonkatheter gelingt es, die unterschiedlichen, endo- und extrakranialen, arterienvenösen Aneurysmen mit dem Erfolg auszuschalten. Zur Zeit stehen die großen und mehrjährigen, positiven Erfahrungen der Behandlung der arteriellen Aneurysmen durch den endovaskulären Eingriff zur Verfügung.

Darüber hinaus werden die erfindungsgemäßen, okkludierenden Vorrichtungen bei der Behandlung der karotidenkavernösen Anastomosen sowie der arteriellen Sinusanastomosen, zur Blockierung der Gefäße von reich vaskularisierten Me- ningiomen weitgehend ausgenutzt. Diese Vorrichtungen sind bei der Okklusion der unterschiedlichen Arterien der inneren Organe, der Hals- und Gehirngefäße erfolgreich ausgenutzt worden.

Die erfindungsgemäßen, okkludierenden Vorrichtungen geben die Möglichkeit, eine zuverlässige Prophylaxe der Blutergüsse infolge der eventuellen, wiederholten Zerreißun- gen der Aneurysmen zu erreichen, praktisch die Jandungsrup- turen der Aneurysmen, Gefäße, das Losreißen der Gefäße von den Organen und Geweben auszuschließen.

PATENTANSPRÜCHE:

1. Okkludierende Vorrichtung, die einen abnehmbaren, aufblasbaren Ballon (1) mit der röntgenkontrasten Marke (2) vorsieht, der vermittels des Sphinkters (3) mit einem einsetzbaren Katheter (4) verbunden ist, d a d u r c h  g e k e n n z e i c h n e t , daß die Wandung des Ballons (1) mindestens eine ringförmige, an dem Sphinkter ( 3 ) herum angebrachte Verdickung (5) trägt.

2. Okkludierende Vorrichtung nach Anspruch 1, d a - d u r c h  g e k e n n z e i c h n e t , daß der Ballon (1) noch mindestens eine ringförmige Zusatzverdickung (12) aufweist.

3. Okkludierende Vorrichtung nach Anspruch 1, 2, d a d u r c h  g e k e n n z e i c h n e t , daß der Ballon (1) mit zumindest einer abgeschlossenen Zelle (8) versehen ist, die mit einem Klebestoff (10) ausgefüllt ist und in ihrer Außenwandung ausgesparte Perforation (9) besitzt.

4. Okkludierende Vorrichtung nach Anspruch 1, 2, d a d u r c h  g e k e n n z e i c h n e t , daß auf der Oberfläche des Ballons (1) zumindest eine zusätzliche Festhalterung begünstigende Zitze (14) angebracht ist, die als Hohlvorsprung mit den Seitenöffnungen (15) ausgeführt ist.

5. Okkludierende Vorrichtung nach einem beliebigen vorhergehenden Anspruch 1 bis 4, d a d u r c h  g e - k e n n z e i c h n e t , daß der Katheter (4) als Abzweigkatheter ausgeführt ist.

FIG.2

FIG.1

FIG.3

FIG.4

FIG.5

FIG.6

FIG.7

FIG.8

FIG.9

FIG.10

FIG.11

# INTERNATIONAL SEARCH REPORT

International Application No PCT/SU 88/00021

| I. CLASSIFICATION OF SUBJECT MATTER (if several classification symbols apply, indicate all) [6] |
|---|

According to International Patent Classification (IPC) or to both National Classification and IPC

IPC[4]  A61M 25/00

| II. FIELDS SEARCHED |
|---|

Minimum Documentation Searched [7]

| Classification System | Classification Symbols |
|---|---|
| IPC[4] | A61M 25/00 |

Documentation Searched other than Minimum Documentation
to the Extent that such Documents are Included in the Fields Searched [8]

| III. DOCUMENTS CONSIDERED TO BE RELEVANT [9] | | |
|---|---|---|
| Category [*] | Citation of Document, [11] with Indication, where appropriate, of the relevant passages [12] | Relevant to Claim No. [13] |
| A | FR, A1, 2361124 (PEVSNER Paul Hershel) 10 March 1978 (10.03.78) see page 4, lines 8-14, figures 12,13; page 9, lines 10-23 (cited in the description) | 3 |
| A | FR, A1, 2383673 (Docteur DEBRUN Gérard) 13 October 1978 (13.10.78) see page 2, lines 23,24; figure 2, page 3, lines 27-35 (cited in the description) | 5 |
| A | SU, A1, 810246 (Kievsky nauchno--issledovatelsky institut neirokhirur-gii) 10 March 1981 (10.03.81) see column 2, lines 5-30, figures 1,2 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

| IV. CERTIFICATION |
|---|

| Date of the Actual Completion of the International Search | Date of Mailing of this International Search Report |
|---|---|
| 26 December 1988 (26.12.88) | 01 March 1989 (01.03.89) |

| International Searching Authority | Signature of Authorized Officer |
|---|---|
| ISA/SU | |

Form PCT/ISA/210 (second sheet) (January 1985)